# FASCICULE DE BREVET EUROPEEN

(11) **EP 3 852 646 B1**
(45) Date de publication et mention de la délivrance du brevet: **13.11.2024**
(21) Numéro de dépôt: 19794181.8
(22) Date de dépôt: 19.09.2019
(51) Int. Cl.: A61B 17/02, A61F 2/46

(54) **OUTIL D'ÉCARTEMENT DE TISSUS MOUS COUVRANT DES VERTÈBRES LOMBAIRES**
WERKZEUG ZUM SPREIZEN VON WEICHTEILEN, DIE DIE LENDENWIRBEL ABDECKEN
TOOL FOR SPREADING SOFT TISSUES COVERING THE LUMBAR VERTEBRAE

(30) Priorité: 21.09.2018 FR 1858595
(43) Date de publication de la demande: 28.07.2021
(73) Titulaire: Allyon, 42100 Saint-Etienne (FR)
(72) Inventeur: SIMON, Lionel, 69360 Solaize (FR)
(74) Mandataire: Germain Maureau
(86) Numéro de dépôt international: PCT/FR2019/052198
(87) Numéro de publication internationale: WO 2020/058644

(56) Documents cités:
- EP-A1- 2 952 140
- US-A1- 2006 106 398
- US-A1- 2010 076 502
- US-A1- 2015 366 549
- US-A1- 2017 000 527
- US-A1- 2018 085 105
- US-B2- 7 166 073
- US-B2- 8 801 626

## Description

La présente invention concerne un outil d'écartement des tissus mous couvrant des vertèbres lombaires, pour des opérations chirurgicales lombaires.

Certaines opérations chirurgicales lombaires nécessitent un outil d'écartement des tissus mous, qui après une incision de la peau et de ces tissus, permet de prendre appui sur une partie de la vertèbre se trouvant au fond de l'incision, puis de faire basculer cet outil afin d'écarter les tissus se trouvant sur un côté de cette incision, afin d'accéder à la vertèbre pour y réaliser l'opération.

Un type d'outil d'écartement connu, présenté notamment par le document WO-A1-20155104507, comporte dans un plan principal une partie tubulaire de guidage recevant en partie arrière un manche de manipulation, et en partie avant une fourche présentant deux points d'appui disposés perpendiculairement à ce plan principal, pour prendre appui sur une vertèbre lombaire, en particulier sur une apophyse transverse de vertèbre.

Après avoir incisé les tissus mous on descend la fourche pour appliquer ses deux pointes sur la vertèbre, ce qui permet uniquement de localiser le point d'entrée d'un outil de perçage d'un trou pilote, et de faire le passage à travers les tissus mous pour une vis pédiculaire venant se serrer dans le pédicule de la vertèbre.

Cet outil d'écartement permet avec une technique peu invasive de localiser et de perforer le point d'entrée pédiculaire pour placer une vis, mais ne permet aucun autre travail sur les structures environnantes nécessitant un décalage latéral ou périphérique des tissus, par exemple pour réaliser une greffe sur l'apophyse transverse ou pour placer une cage intervertébrale.

De plus cet outil d'écartement est en contact avec l'apophyse articulaire qui peut être déformée par l'arthrose. Il en résulte que sa géométrie contrainte le rend inadapté à toutes les anatomies, au risque d'imposer un mauvais trajet de l'outil de perçage.

Par ailleurs en appliquant la fourche de contact sur la jonction de l'apophyse transverse et de l'apophyse articulaire, l'outil d'écartement occulte alors cette apophyse transverse ce qui empêche d'y accéder, par exemple pour déposer de la greffe.

L'état de la technique peut également être illustré par les enseignements des documents antérieurs US7166073 et US2017/000527 qui proposent chacun un écarteur pour effectuer une chirurgie de la colonne vertébrale qui épargne les muscles lombaires de toute perturbation chirurgicale.

Il est également connu du document EP2952140 d'employer un écarteur de plaque vertébrale constitué d'une poignée à laquelle est solidarisé un corps d'écarteur consistant en une barre métallique en forme de L dont l'extrémité distale s'incurve vers la direction de la poignée.

La présente invention a notamment pour but d'éviter ces inconvénients de la technique antérieure.

Elle propose à cet effet un outil d'écartement de tissus mous selon la revendication 1.

Un avantage de cet outil d'écartement est que la plaque d'appui constituant un plan parallèle au plan principal, occupe dans la direction transversale un espace réduit égal à l'épaisseur de ce plan, et est préférentiellement disposée dans le prolongement d'un bord longitudinal de l'écarteur. De cette manière on dégage l'espace en avant de l'écarteur, permettant d'y effectuer des opérations chirurgicales. On fournit également une surface d'appui sur la face externe de la plaque pour guider un geste d'arthrectomie ou d'insertion d'une cage intervertébrale.

L'outil d'écartement selon l'invention peut de plus comporter une ou plusieurs des caractéristiques suivantes, qui peuvent être combinées entre elles.

Avantageusement, le contour d'accrochage de la plaque d'appui est orienté vers le côté face. De plus, ce contour d'accrochage peut comporter des dents d'accrochage sur la vertèbre. On réalise un accrochage plus sûr de l'outil.

Avantageusement, la plaque d'appui comporte vers l'arrière une butée saillante tournée vers le côté face. Cette butée venant sur la vertèbre, limite la descente de l'outil dans le patient.

Avantageusement, l'écarteur présente dans sa section transversale une forme bombée tournée vers le côté dos.

Selon l'invention, l'outil d'écartement comporte une distance longitudinale entre l'extrémité avant de la plaque d'appui et l'extrémité transversale avant de l'écarteur, qui est supérieure à six millimètres. On dégage de cette manière un espace en avant de l'écarteur, pour y effectuer des opérations.

Selon un mode de réalisation, l'outil d'écartement comporte deux plaques d'appui sensiblement parallèles, disposées symétriquement sur les bords longitudinaux de l'écarteur.

Dans ce cas, l'outil peut comporter un pontet reliant les deux plaques d'appui.

Selon un autre mode de réalisation, l'outil d'écartement comporte deux écarteurs disposés transversalement de chaque côté de la plaque d'appui.

Avantageusement, l'écarteur comporte des repères radiologiques.

En particulier, l'outil peut comporter un trait longitudinal formant un repère radiologique.

En complément, l'outil d'écartement peut comporter un repère présentant une dissymétrie par rapport au plan médian de l'écarteur.

L'invention sera mieux comprise et d'autres caractéristiques et avantages apparaîtront plus clairement à la lecture de la description ci-après donnée à titre d'exemple, en référence aux dessins annexés dans lesquels :
- la figure 1 présente un outil d'écartement selon l'invention ;
- les figures 2a et 2b présentent l'avant de l'outil, respectivement en vue de face et en coupe suivant le plan de coupe médian IIb-IIb ;
- les figures 3 et 4 présentent la mise en place de cet outil sur une apophyse articulaire et en appui contre la face latérale de l'apophyse transverse ;
- la figure 5 présente le basculement de cet outil pour écarter les tissus mous se trouvant au dos de l'écarteur ;
- les figures 6 et 7 présentent l'insertion d'une cage dans l'espace intervertébral après le basculement de cet outil, la figure 7 montrant la portion d'apophyse articulaire en saillie sur le trajet de la cage, qui peut être idéalement réséquée grâce au guidage de la plaque d'écarteur pour libérer ce trajet ;
- la figure 8 présente l'implantation d'une vis à côté de la cage ;
- les figures 9 et 10 présentent en variante un outil d'écartement à doubles écarteurs symétriques par rapport à la plaque d'appui ;
- les figures 11 et 12 présentent en variante un outil d'écartement à double plaques d'appui disposées chacune dans le prolongement de l'un des deux bords longitudinaux de l'écarteur, de part et d'autre du bord transversal avant ;
- la figure 13 présente cet outil comprenant un pontet de liaison des deux plaques d'appui ;
- la figure 14 présente en variante un outil d'écartement pourvu d'un repère visuel et radiologique ;
- la figure 15 présente en variante deux outils d'écartement pourvus d'un repère visuel et radiologique permettant sur les radiographies de profil une différenciation de ces outils ; et
- les figures 16 et 17 présentent en variante un outil d'écartement comprenant une plaque d'appui équipée d'une butée.

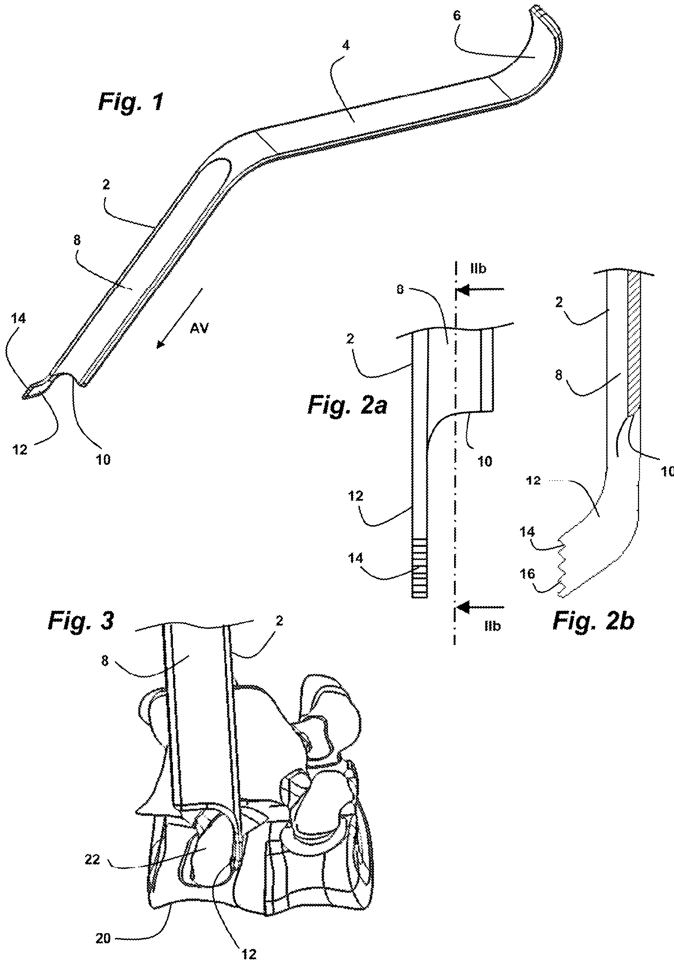

Les figures 1, 2a et 2b présentent un outil d'écartement formé dans cet exemple dans une bande de largeur constante, découpée dans une tôle métallique et mise en forme avec une courbure disposée dans un plan principal, comprenant un écarteur 2 présentant successivement, en partant de l'avant indiqué par la flèche AV, une partie avant rectiligne allongée suivant une direction longitudinale et équipée d'une plaque d'appui 12 plane, puis une partie arrière définissant un manche rectiligne 4 qui se termine par un crochet arrière 6.

Le manche 4 disposant dans cet exemple d'une section transversale plate, forme avec l'écarteur 2 un pli tourné vers un côté appelé dos, présentant un angle d'environ 135°. Le crochet arrière 6 tourné vers le côté face, opposé au côté dos, présente une courbure régulière suivant un angle d'environ 120°.

L'écarteur 2 comporte dans sa section transversale une forme bombée qui est tournée vers le dos, formant sur la face un creux 8, symétrique par rapport au plan médian de l'outil, recevant sur chaque bord une partie plate de largeur réduite. L'extrémité avant de l'écarteur 2 comporte un bord transversal avant 10, qui après une courbure vers l'avant se prolonge par la plaque d'appui 12.

La plaque d'appui 12 constituée par la tôle formant un plan sensiblement parallèle au plan principal, prolonge vers l'avant l'écarteur 2 sur son bord longitudinal de manière à rester sensiblement dans la largeur de la bande. La plaque d'appui 12 remonte vers le côté face, pour se terminer par un contour d'accrochage 14 disposé suivant la direction longitudinale, comprenant cinq pointes 16 réparties sur ce contour d'accrochage 14.

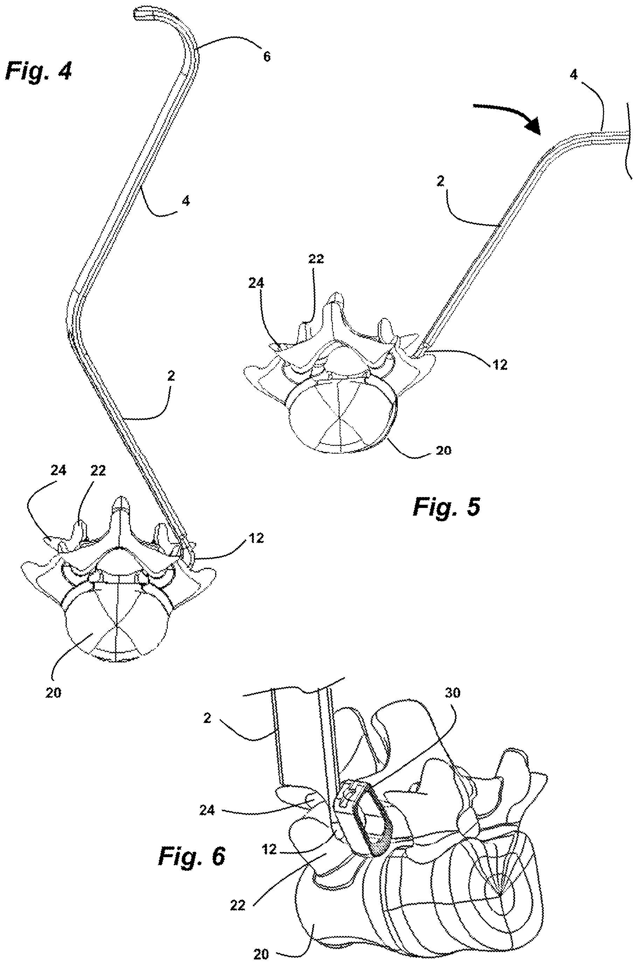

Les figures 3 et 4 présentent après l'incision médiane de la peau et la désinsertion des muscles de la lame postérieure, la descente de l'outil d'écartement sur une vertèbre 20, puis le positionnement du contour d'accrochage 14 à l'extrémité de la plaque d'appui 12 sur une apophyse articulaire 24 et en apposition sur la face latérale de l'apophyse transverse 22, cette plaque étant perpendiculaire à l'axe de la colonne vertébrale.

On notera que la section de l'écarteur 2 avec sa plaque d'appui 12 présente une épaisseur réduite, ce qui permet de pratiquer une incision minimum sur la peau et les tissus pour son introduction. On réalise une chirurgie restreinte qui est peu intrusive, proche de la taille d'incisions mini-invasives réalisées avec l'outil d'écartement suivant l'art antérieur présenté ci-dessus. En particulier, sa configuration géométrique apporte un écartement profond et précis améliorant l'exposition par rapport aux habitudes chirurgicales utilisant notamment un écarteur de Beckmann, créant ainsi une chirurgie dont l'invasivité est intermédiaire entre la chirurgie ouverte et la chirurgie mini-invasive présentée dans l'art antérieur ci-dessus.

Le crochet arrière 6 du manche 4 permet le calage de la main du chirurgien ou l'accrochage d'un lien tendu pour maintenir l'inclinaison de l'écarteur 2, ou en variante l'accrochage d'un cadre maintenant la distance entre deux écarteurs disposés symétriquement. De plus le crochet 6 peut être centré sur le plan médian, ou décalé latéralement pour compenser un décalage des efforts donnés par la plaque d'appui 12 disposée le long du bord longitudinal.

La figure 5 présente, après l'appui des pointes 16 du contour d'accrochage 14 sur l'apophyse articulaire 24 de manière à accrocher solidement la plaque d'appui 12, un basculement vers le dos de l'outil d'écartement. La forme bombée sur le dos de l'écarteur 2 repousse les tissus mous sans les blesser, de manière à dégager un espace de travail vers le plan médian du dos du patient.

La forme allongée de l'outil d'écartement, comprenant le manche 4 et son crochet arrière 6 facilitant la prise en main de l'outil et donnant au chirurgien un repère sur ce manche 4, constitue un bras de levier important permettant d'appliquer une pression suffisamment élevée sur les tissus pour les écarter, tout en gardant un appui des pointes 16 sur la vertèbre 20 afin d'éviter son glissement.

On obtient en particulier avec l'outil selon l'invention plusieurs géométries d'intérêt. On peut appliquer la plaque d'appui 12 contre la face latérale de l'apophyse articulaire 24, cette face servant de pivot de l'outil pour provoquer l'écartement. On peut aussi appliquer la plaque d'appui 12 contre la face crânienne de l'apophyse transverse associée au pédicule à protéger en cas d'arthrectomie, comprenant la découpe de l'apophyse articulaire permettant en particulier de libérer de l'espace afin de passer une cage intervertébrale.

De plus la plaque d'appui 12 se trouvant dans le prolongement du bord longitudinal de l'écarteur 2, et le bord transversal avant 10 de l'écarteur 2 étant dans la direction longitudinale éloigné de la plaque d'appui 12, on obtient un dégagement entre cet écarteur 2 et l'apophyse transverse 22. Ce dégagement évite un contact précoce avec la face postérieure de l'apophyse transverse 22 pour permettre son exposition, afin de déposer de la greffe si nécessaire, et d'éviter une fracture de cette apophyse transverse par le mouvement de pivotement.

On obtient aussi un accès précis en face de cet écarteur devant sa face transversale avant 10 qui dégage toutes les structures d'intérêt pour la chirurgie vertébrale, à savoir la lame postérieure, le point d'entrée pédiculaire, l'apophyse articulaire, la face postérieure de l'apophyse transverse et, sur le côté, l'espace discal intervertébral.

Avantageusement la distance longitudinale entre l'extrémité avant de la plaque d'appui 12 et l'extrémité transversale avant 10 de l'écarteur 2, est supérieure à six millimètres, soit au moins le rayon d'une vis à implanter, et peut atteindre vingt millimètres.

On obtient un accès à la lame postérieure utile en cas de canal lombaire étroit, un accès au trajet pédiculaire nécessaire pour placer des vis, un accès à l'espace intervertébral pour le placement d'une cage intervertébrale, et un accès à la face postérieure de l'apophyse transverse pour la greffe postéro-latérale.

L'outil écartement formé d'une seule pièce dans un matériau inaltérable, ne comprenant pas de parties en mouvement comme une articulation une crémaillère, est inusable. Il peut être utilisé en alternance d'un côté puis de l'autre, en permettant un accès plus précis, en profondeur, avec une stabilité qui libère intégralement l'espace de travail.

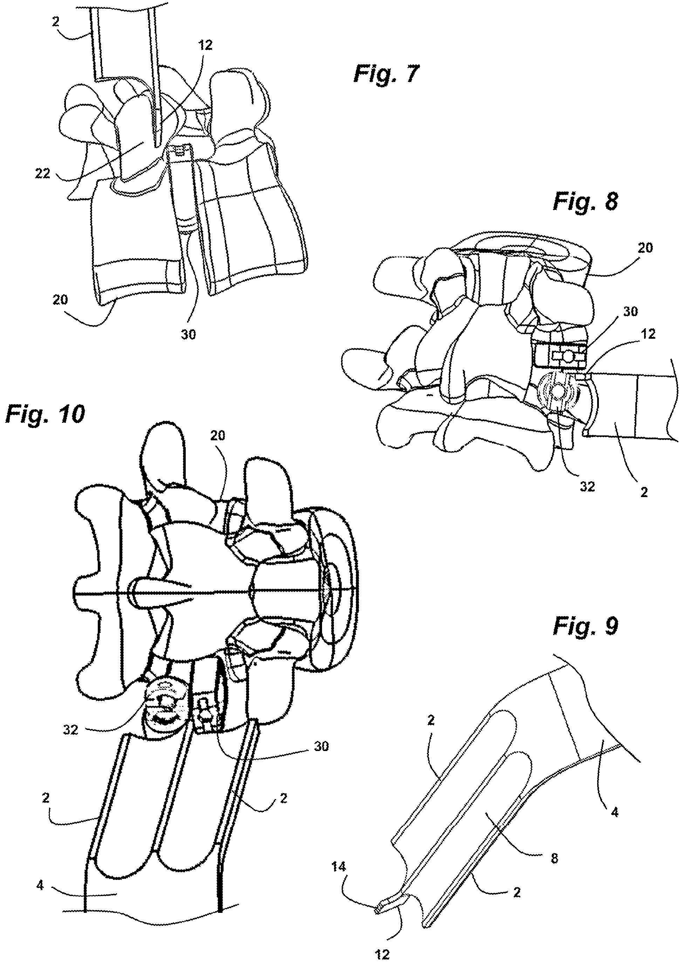

Les figures 6 et 7 présentent le côté extérieur de la plaque d'appui 12 formant une direction du trait de coupe de l'apophyse articulaire 24, permettant ensuite la pénétration d'une cage intervertébrale 30 dans cette découpe, le long de la plaque d'appui.

La figure 8 présente la mise en place d'une vis 32 sur le côté de la plaque d'appui 12 opposé à la cage 30, cette plaque d'appui 12 formant une séparation entre les deux espaces implantaires.

Les figures 9 et 10 présentent un outil d'écartement comportant deux écarteurs parallèles 2 comprenant chacun une forme bombée, disposés symétriquement par rapport à la plaque d'appui 12 se trouvant entre ces deux écarteurs 2.

Les deux écarteurs 2 parallèles permettent à partir d'un appui central de la plaque d'appui 12, d'écarter les tissus mous se trouvant de chaque côté de cette plaque d'appui 12. On peut ainsi par exemple accéder simultanément à une cage 30 et à une vis 32, en disposant la plaque d'appui 12 entre ces deux éléments.

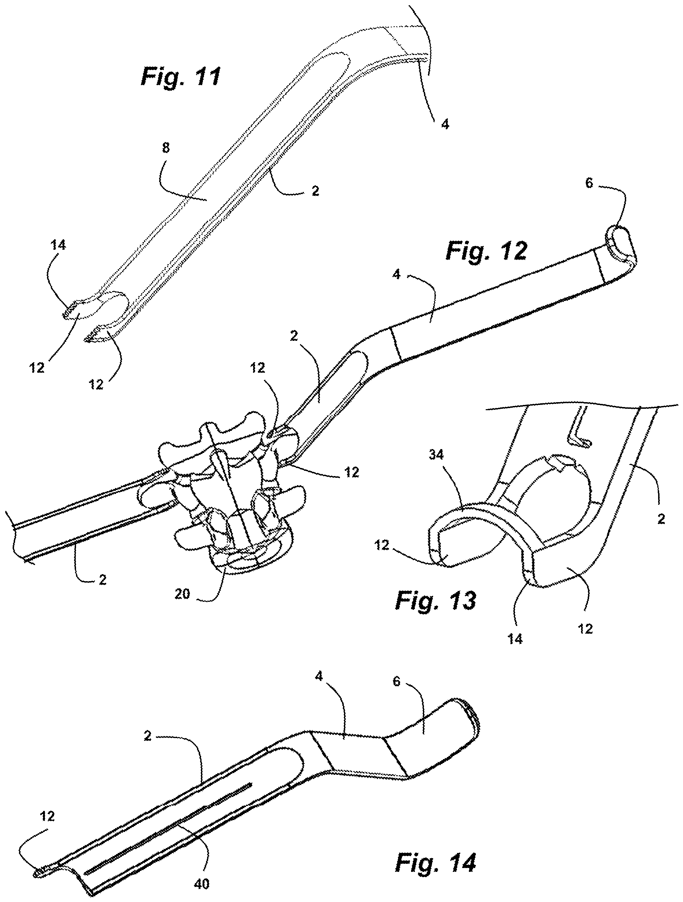

Les figures 11 et 12 présentent un outil d'écartement comprenant un unique écarteur 2, comportant deux plaques d'appui 12 parallèles disposées symétriquement sur les bords longitudinaux de cet écarteur.

Cet outil écartement étant symétrique, un même outil peut être utilisé à gauche et à droite comme présenté figure 12. En particulier la distance transversale entre les deux plaques d'appui 12 doit être suffisante pour permettre les opérations entre elles. On prévoit avantageusement une distance comprise entre cinq et vingt-cinq millimètres.

La figure 13 présente en variante un outil d'écartement avec deux plaques d'appui 12 parallèles, reliées à leurs extrémités avant par une jonction étroite formant un pontage bombé 34 tourné vers le côté face, ce qui rigidifie fortement ces plaques d'appui 12.

La figure 14 présente un outil d'écartement comportant un trait longitudinal 40 formant un repère visuel et radiologique, marqué dans le creux 8 de l'écarteur 2 suivant son axe de symétrie. En particulier le repère visuel peut être positionné à une distance telle de la plaque d'appui 12 qu'il prolonge l'axe du pédicule vertébral lorsque cette plaque d'appui est apposée contre la face latérale de l'apophyse transverse 22, dont la forme est quasi invariable d'un patient à l'autre, soit à une distance d'environ 9mm.

Le trait longitudinal 40 constitue un repère prolongeant le trajet pédiculaire envisagé a priori par le chirurgien, il permet une double identification visuelle et radiologique. On peut ainsi corriger la trajectoire finale de l'outil de perçage par rapport à la trajectoire indiquée par le repère, après visualisation de l'outil d'écartement en position sur la structure osseuse du patient par des clichés radiologiques pendant l'opération. Cela garantit un placement optimal de la vis pédiculaire.

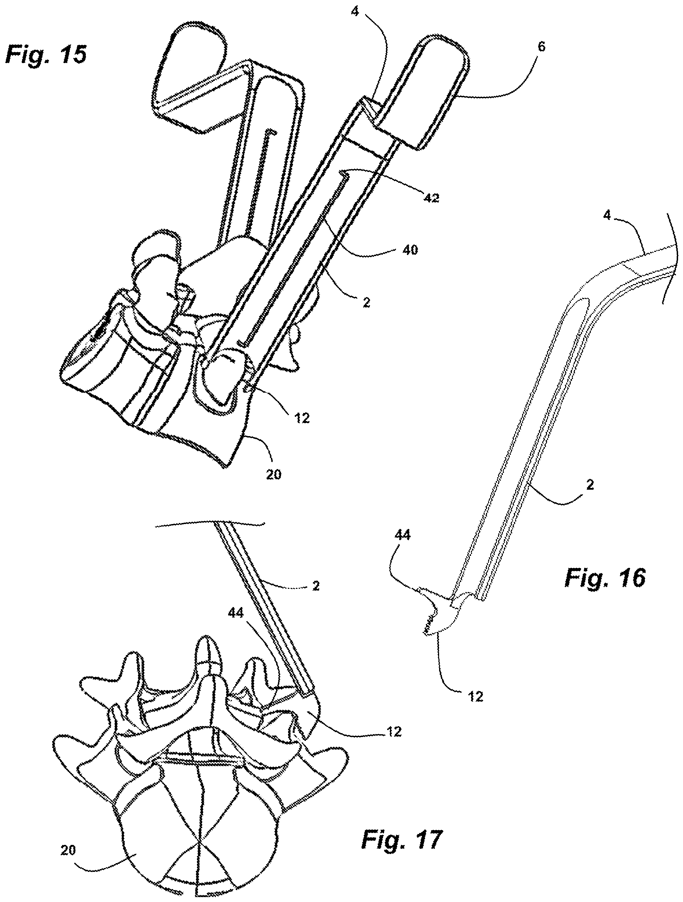

La figure 15 présente un outil d'écartement comprenant à chaque extrémité du trait longitudinal 40 formant le repère, un petit trait transversal 42, ces deux petits traits tournés transversalement du même côté par rapport au plan médian pédiculaire, formant un repère non symétrique.

Lors de l'utilisation de deux outils d'écartement en opposition comme présenté sur la figure 15, on obtient par un effet de symétrie sur ces deux outils des traits transversaux 42 tournés de deux côtés opposés. Ce retournement de direction de ces traits transversaux 42 forme un marquage différenciant les deux outils d'écartement utilisés, donnant un repère visuel sur une radiographie permettant d'identifier sans erreur chaque outil.

En particulier le trait longitudinal 40 et les traits perpendiculaires 42 peuvent être réalisées par une fente dans un matériau opaque aux rayonnements radiologiques.

Les figures 16 et 17 présentent un outil d'écartement comprenant à l'arrière de la plaque d'appui 12, un bec formant une butée saillante 44 restant dans le plan de cette plaque, qui pointe vers le côté face, perpendiculairement à l'axe longitudinal de l'écarteur 2.

La butée saillante 44 constitue lors d'un mouvement de bascule de l'outil d'écartement, un appui empêchant cet outil de plonger trop profondément sous le niveau de la transverse.

D'une manière générale l'outil écartement peut être réalisé avec toutes matières métalliques, plastiques ou autres, qui sont radio-transparentes ou radios-opaques. Ces outils peuvent être à usage unique, ou réutilisables après une stérilisation.

## Revendications

1. - Outil d'écartement de tissus mous couvrant des vertèbres lombaires, formé dans une bande mise en forme avec une courbure disposée dans un plan principal, ledit outil d'écartement comportant un écarteur (2) présentant un côté dos prévu pour faire face aux tissus mous, un côté face opposé audit côté dos et deux bords longitudinaux, cet écarteur (2) comportant une partie arrière formant un manche (4), et une partie avant rectiligne allongée suivant une direction longitudinale et présentant une extrémité avant conçue pour servir d'appui sur une vertèbre (20) afin d'effectuer un pivotement dans ledit plan principal autour d'un axe transversal, perpendiculaire à ce plan principal, pour appliquer le côté dos de l'écarteur (2) sur les tissus mous afin de les repousser, dans lequel la partie avant de l'écarteur (2) comprend au moins un bord transversal avant (10) qui, après une courbure vers l'avant, se prolonge par une plaque d'appui (12), ladite plaque d'appui (12) étant plane dans un plan sensiblement parallèle au plan principal prolongeant ainsi vers l'avant l'écarteur (2) sur son bord longitudinal de manière à rester sensiblement dans la largeur de la bande, et la plaque d'appui (12) remontant vers le côté face pour se terminer par un contour d'accrochage (14) disposé suivant la direction longitudinale ;
ledit outil d'écartement étant **caractérisé en ce qu'**il comporte une distance longitudinale entre une extrémité avant de la plaque d'appui (12) et le bord transversal avant (10) de l'écarteur (2), qui est supérieure à six millimètres.

2. - Outil d'écartement selon la revendication 1, **caractérisé en ce que** le contour d'accrochage de la plaque d'appui est orienté vers le côté face.

3. - Outil d'écartement selon la revendication 1 ou 2, **caractérisé en ce que** le contour d'accrochage (14) de la plaque d'appui (12) comporte des dents d'accrochage (16) sur la vertèbre (20).

4. - Outil d'écartement selon l'une quelconque des revendications précédentes, **caractérisé en ce que** la plaque d'appui (12) comporte vers l'arrière une butée saillante (44) tournée vers le côté face.

5. - Outil d'écartement selon l'une quelconque des revendications précédentes, **caractérisé en ce que** l'écarteur (2) présente dans sa section transversale une forme bombée tournée vers le côté dos.

6. - Outil d'écartement selon l'une quelconque des revendications précédentes, **caractérisé en ce qu'**il comporte deux plaques d'appui (12) sensiblement parallèles, disposées symétriquement sur les bords longitudinaux de l'écarteur (2).

7. - Outil d'écartement selon la revendication 6, **caractérisé en ce qu'**il comporte un pontet (34) reliant les deux plaques d'appui (12).

8. - Outil d'écartement selon l'une quelconque des revendications 1 à 5, **caractérisé en ce qu'**il comporte deux écarteurs (2) disposés transversalement de chaque côté de la plaque d'appui (12).

9. - Outil d'écartement selon l'une quelconque des revendications précédentes, **caractérisé en ce que** l'écarteur (2) comporte des repères radiologiques.

10. - Outil d'écartement selon la revendication 9, **caractérisé en ce qu'**il comporte un trait longitudinal (40) formant un repère radiologique.

11. - Outil d'écartement selon la revendication 9 ou 10, **caractérisé en ce qu'**il comporte un repère présentant une dissymétrie (42) par rapport au plan médian de l'écarteur (2).

## Patentansprüche

1. Werkzeug zum Spreizen von Weichgewebe, das Lendenwirbel bedeckt, ausgebildet aus einem Band, das mit einer in einer Hauptebene angeordneten Krümmung gebildet ist, wobei das Spreizwerkzeug einen Spreizer (2) umfasst, der eine Rückseite, die so vorgesehen ist, dass sie dem Weichgewebe zugewandt ist, eine der Rückseite gegenüberliegende Vorderseite und zwei Längskanten aufweist, wobei der Spreizer (2) einen hinteren Teil, der einen Griff (4) bildet, und einen geraden vorderen Teil aufweist, der sich in eine Längsrichtung erstreckt und ein vorderes Ende aufweist, das so ausgelegt ist, dass es als Stütze für einen Wirbel (20) dient, um in der Hauptebene um eine Querachse, die die senkrecht zu der Hauptebene verläuft, eine Schwenkung auszuführen, um die Vorderseite des Spreizers (2) an das Weichgewebe anzulegen, um es zurückzuschieben, wobei der vordere Teil des Spreizers (2) mindestens eine vordere Querkante (10) umfasst, die sich nach einer Krümmung nach vorne in eine Stützplatte (12) verlängert, wobei die Stützplatte (12) flach in einer Ebene ist, die im Wesentlichen parallel zur Hauptebene verläuft, und den Spreizer (2) an seiner Längskante so nach vorne verlängert, dass er im Wesentlichen innerhalb der Breite des Bandes bleibt, und die Stützplatte (12) sich zur Rückseite hin erhöht, um in einer in der Längsrichtung angeordneten Einhakkontur (14) zu enden;
wobei das Spreizwerkzeug **dadurch gekennzeichnet ist, dass** es einen Längsabstand zwischen einem vorderen Ende der Stützplatte (12) und der vorderen Querkante (10) des Spreizers (2) umfasst, der größer als sechs Millimeter ist.

2. Spreizwerkzeug nach Anspruch 1, **dadurch gekennzeichnet, dass** die Einhakkontur der Stützplatte zur Vorderseite hin ausgerichtet ist.

3. Spreizwerkzeug nach Anspruch 1 oder 2, **dadurch gekennzeichnet, dass** die Einhakkontur (14) der Stützplatte (12) Einhakzähne (16) am Wirbel (20) umfasst.

4. Spreizwerkzeug nach einem der vorhergehenden Ansprüche, **dadurch gekennzeichnet, dass** die Stützplatte (12) nach hinten einen der Vorderseite zugewandten vorspringenden Anschlag (44) umfasst.

5. Spreizwerkzeug nach einem der vorhergehenden Ansprüche, **dadurch gekennzeichnet, dass** der Spreizer (2) in seinem Querschnitt eine der Rückseite zugewandte gewölbte Form aufweist.

6. Spreizwerkzeug nach einem der vorhergehenden Ansprüche, **dadurch gekennzeichnet, dass** es zwei im Wesentlichen parallele Stützplatten (12) umfasst, die symmetrisch an den Längskanten des Spreizers (2) angeordnet sind.

7. Spreizwerkzeug nach Anspruch 6, **dadurch gekennzeichnet, dass** es eine Brücke (34) umfasst, die die beiden Stützplatten (12) verbindet.

8. Spreizwerkzeug nach einem der Ansprüche 1 bis 5, **dadurch gekennzeichnet, dass** es zwei quer auf jeder Seite der Stützplatte (12) angeordnete Spreizer (2) umfasst.

9. Spreizwerkzeug nach einem der vorhergehenden Ansprüche, **dadurch gekennzeichnet, dass** der Spreizer (2) radiologische Markierungen umfasst.

10. Spreizwerkzeug nach Anspruch 9, **dadurch gekennzeichnet, dass** es eine Längslinie (40) umfasst, die eine radiologische Markierung bildet.

11. Spreizwerkzeug nach Anspruch 9 oder 10, **dadurch gekennzeichnet, dass** es eine Markierung umfasst, die eine Asymmetrie (42) in Bezug auf die Mittelebene des Spreizers (2) aufweist.

## Claims

1. A tool for retracting soft tissue covering lumbar vertebrae, formed in a strip shaped with a curvature disposed in a main plane, said retracting tool including a retractor (2) having a back side designed to face the soft tissue, a face side opposite said back side and two longitudinal edges, this retractor (2) including a rear portion forming a handle (4), and a rectilinear front portion elongated in a longitudinal direction and having a front end designed to act as a support on a vertebra (20) in order to perform a pivoting in said main plane about a transverse axis, perpendicular to this main plane, to apply the back side of the retractor (2) on the soft tissue in order to push them back, wherein the front portion of the retractor (2) comprises at least one front transverse edge (10) which, after bending forward, is extended by a support plate (12), said support plate (12) being flat in a plane substantially parallel to the main plane so extending the retractor (2) forwardly on its longitudinal edge so as to remain substantially in the width of the strip, and the support plate (12) rising towards the face side to end with a hooking contour (14) arranged in the longitudinal direction;
said retracting tool being **characterized in that** it includes a longitudinal distance between a front end of the support plate (12) and the front transverse edge (10) of the retractor (2), which is greater than six millimeters.

2. The retracting tool according to claim 1, **characterized in that** the hooking contour of the support plate is oriented towards the face side.

3. The retracting tool according to claim 1 or 2, **characterized in that** the hooking contour (14) of the support plate (12) includes hooking teeth (16) on the vertebra (20).

4. The retracting tool according to any one of the preceding claims, **characterized in that** the support plate (12) includes towards the rear a projecting stop (44) facing the face side.

5. The retracting tool according to any one of the preceding claims, **characterized in that** the retractor (2) has in its cross section a convex shape facing the back side.

6. The retracting tool according to any one of the preceding claims, **characterized in that** it includes two support plates (12) substantially parallel, disposed symmetrically on the longitudinal edges of the retractor (2).

7. The retracting tool according to claim 6, **characterized in that** it includes a bridge (34) connecting the two support plates (12).

8. The retracting tool according to any one of claims 1 to 5, **characterized in that** it includes two retractors (2) disposed transversely on each side of the support plate (12).

9. The retracting tool according to any one of the preceding claims, **characterized in that** the retractor (2) includes radiological marks.

10. The retracting tool according to claim 9, **characterized in that** it includes a longitudinal line (40) forming a radiological mark.

11. The retracting tool according to claim 9 or 10, **characterized in that** it includes a mark having an asymmetry (42) relative to the median plane of the retractor (2).
